# EUROPEAN PATENT SPECIFICATION

(11) **EP 1 590 574 B1**
(45) Date of publication and mention of the grant of the patent: **21.03.2007**
(21) Application number: 04703181.0
(22) Date of filing: 19.01.2004
(51) Int. Cl.: F16B 21/18, F16B 39/00, F16B 2/20, A61M 15/00, B65D 83/14

(54) **FIXATION DEVICE**
BEFESTIGUNGSVORRICHTUNG
DISPOSITIF DE FIXATION

(30) Priority: 21.01.2003 GB 0301366
(43) Date of publication of application: 02.11.2005
(73) Proprietor: GLAXO GROUP LIMITED, Greenford, Middlesex UB6 ONN (GB)
(72) Inventor: GODFREY, James W., c/o GlaxoSmithKline, Hertfordshire SG12 0DP (GB)
(74) Representative: Rice, Jason Neale
(86) International application number: PCT/EP2004/000663
(87) International publication number: WO 2004/065224

(56) References cited:
- WO-A-01/28887
- DE-C- 887 716
- US-A- 1 638 209
- US-A- 3 853 413
- "WALDES TRUARC RETAINING RING" MACHINE DESIGN, PENTON,INC. CLEVELAND, US, vol. 61, no. 1, 12 January 1989 (1989-01-12), page 161 XP000045387 ISSN: 0024-9114

## Description

### Field of the Invention

The present invention relates to a fixation device which is for fixing together two parts of a multi-part assembly and which has a ring-like body, hereinafter a "fixation device of the type defined".

### Background of the Invention

A fixation device of the type defined is disclosed in WO-A-0128887 (Glaxo/Brand *et al*) for connecting a device housing to the business end of an aerosol canister. The device housing is for housing a mechanical or electrical device which is to be used with the canister, for instance an actuation indicator, such as of the type disclosed in WO-A-9856444 (Glaxo/Rand *et al*) or a sensor.

As shown in FIGURES 1a and 1b of the accompanying drawings, the fixation device 1 of WO-A-0128887 has a ring-like body 3 of generally C-shape due to the body 3 having an axial split 5. The fixation device 1 is integrally formed with the split 5 in the body 3. However, when the fixation device 1 is mass produced the fixation devices 1 tend to become entangled with one another on the production line (e.g. in bowl feeders) due to them being integrally formed with the split 5 in the body 3.

The aim of the present invention is to provide a fixation device of the type defined which alleviates the problem of entanglement.

### Summary of the Invention

According to a first aspect of the present invention there is provided a fixation device according to claim 1.

Thus, the problem of entanglement is overcome due to the body being manufactured with an endless angular extent. The split is formed at a later stage through provision of the weakened zone, for instance after a bowl feeder step in an automated production line process.

To move the body from the manufactured state to the use state, the weakened zone may be such that the split is formed by applying a radial force on the body, e.g. a radially outward (tensile) force or a radially inward (compressive) force.

In an embodiment of the invention, such as hereinafter described, the fixation device is of a plastics material, for instance made by a moulding process, such as injection moulding or the like.

In an embodiment of the invention, such as hereinafter described, the fixation device is integrally formed with the weakened zone. For instance, the fixation device is moulded with the weakened zone formed therein.

The fixation device may consist of the ring-like body.

The weakened zone may be a structural discontinuity in the body, e.g. a notch, or a frangible seam, etc..

Preferably, the body has a generally C-shape in the use state.

Further preferably, the body is of curvilinear form, for instance generally circular. The body may instead have a polygonal shape, e.g. square, hexagonal etc.. Typically, the cross-sectional shape of the body is complementary to the cross-sectional shape of the surfaces of the first and second parts of the assembly between which the fixation device is to be interposed.

Preferably, the split allows the body to be radially expanded when in its use state. More preferably, the body is reversibly radially expandable when in its use state, e.g. the body is resilient so that it has a biasing force which biases the body to a radially contracted configuration. These features enable the fixation device to be custom fitted on one of the assembly parts.

According to a second aspect of the invention there is provided a system comprising a first part of a multi-part assembly, a second part of the assembly and a fixation device according to the first aspect for fixing the first and second parts together.

According to a third aspect of the invention there is provided a system according to the second aspect in which the fixation device is in its use state interposed between the first and second parts to fix them together.

The fixation device is preferably wedged between the first and second parts.

The fixation device may be interposed between an outer surface of the first part and an inner surface of the second part.

The outer surface of the first part is preferably a re-entrant surface. More preferably, the re-entrant surface is presented by a neck or waist of the first part, in other words the outer surface is a flaring or tapering surface.

In an alternative embodiment, the inner surface of the second part is a re-entrant surface, e.g. a flaring or tapering surface.

Further preferably, the inner surface is presented by a skirt of the second part which extends about the outer surface of the first part.

Preferably, the first part has a longitudinal axis and the fixation device prevents removal of the second part from the first part in a first axial direction. To this end, the outer surface of the first part may flare/taper laterally outwardly in the first direction. In the alternative embodiment, the inner surface of the second part may flare/taper laterally inwardly in the first direction.

The first and second parts may each have an abutment surface in abutting relation to prevent the second part being removed from the first part in a second axial direction. The abutment surface of the first part is thus axially spaced from the outer surface in the first direction, and may be an end surface of the first part. The inner surface of the second part may be a first inner surface with the abutment surface of the second part being a second inner surface which extends laterally to the first inner surface.

Typically, the ring-like body has radially spaced-apart inner and outer circumferential surfaces. Preferably, the inner circumferential surface of the body bears against the outer surface of the first part and the outer circumferential surface bears against the inner surface of the second part. More preferably, the inner circumferential surface has a complementary profile to that of the outer surface of the first part.

Preferably, the outer circumferential surface of the body is conjoined to the inner surface of the second part, e.g. through a joint formed, for instance, by adhesive, a weld or the like. An ultrasonic weld is particularly preferred. Alternatively, or additionally, the inner circumferential surface may be conjoined to the outer surface of the first part. This is particularly preferred where the inner surface, not the outer surface, is a re-entrant surface.

The multi-part assembly may be a product dispenser with the first part a product container. The second part may be an accessory of the dispenser, e.g. in the form of a cap. The second part may have at least a part of an actuator mechanism for actuating the dispenser, or may, as in the exemplary embodiment hereinafter described, be an actuation indicator which indicates actuation of the dispenser, preferably by indicating the number of doses of the product dispensed or left for dispensing (a dose counter). The dispenser may be a medicament dispenser with the container containing a medicament.

According to a fourth aspect of the invention there is provided a method of fixing a first part of a multi-part assembly to a second part thereof which comprises the steps of providing a fixation device according to the first aspect, forming an axial split in the body at the weakened zone and interposing the fixation device between the first and second parts such that the first and second parts are fixed together through the fixation device.

The fixation device may be moved to the use state prior to being interposed between the first and second parts.

The fixation device may be radially expanded to the use state by inserting a tapered structure into the body or by inserting a radially expandable structure into the body and then causing the radially expandable structure to radially expand.

The first part may have a longitudinal axis, a lateral end surface, and a longitudinal side surface which extends towards the end surface in a first axial direction and which has a profile which tapers laterally outwardly in the first axial direction, and the second part has a longitudinal axis, a lateral surface and a longitudinal skirt, wherein the first and second parts are assembled with the axes aligned, the respective lateral surfaces in bearing relation and the skirt spaced laterally from the tapered profile of the longitudinal surface of the first part, and wherein the body of the fixation device is wedged in a radially expanded condition in the space between the skirt and the tapered profile. The body of the fixation device may be conjoined to the skirt and/or the tapered profile, preferably to the skirt.

The first part may have a longitudinal axis, a lateral end surface, and a longitudinal side surface which extends towards the end surface in a first axial direction and which has a re-entrant section, and the second part has a longitudinal axis, a lateral surface and a longitudinal skirt, wherein the first and second parts are assembled with the axes aligned, the respective lateral surfaces in bearing relation to prevent removal of the second part from the first part in a second axial direction opposite to the first axial direction, and the skirt disposed adjacent the re-entrant section of the longitudinal surface of the first part, and wherein the body of the fixation device is conjoined to the skirt and in bearing relation with the re-entrant section to prevent the second part being removed from the first part in the first axial direction.

The second part may be a cap which is slidingly received on the first part.

The first and/or second parts may be of generally cylindrical cross section.

To make it easier to conjoin the body of the fixation device to the second part, the body of the fixation device is preferably made from the same material as the mating surface of the second part.

In accordance with the present invention, features from the different aspects thereof can be incorporated into the other aspects.

Other preferred features of the invention are set forth in the exemplary embodiment of the present invention which will now be described with reference to the accompanying FIGURES of drawings.

### Brief Description of the Figures of Drawings

FIGURE 1a is a perspective view of a prior art fixation device having a ring-like body.

FIGURE 1b is a schematic, fragmentary side view of the prior art fixation device.

FIGURE 2a is a perspective view of a fixation device in accordance with the present invention with the ring-like body in its manufactured state.

FIGURE 2b is a schematic, fragmentary side view of the fixation device of FIGURE 2a.

FIGURE 2c is a plan view of the fixation device of FIGURE 2a.

FIGURE 2d is a cross-sectional side view of the fixation device of FIGURE 2a along line II-II in FIGURE 2c.

FIGURE 2e is a schematic view of the fixation device of FIGURE 2a, but with the ring-like body having been irreversibly moved to its use state.

FIGURE 3 is a schematic side view, partly in cross section, of an assembly of an aerosol canister and a cap accessory fixed to one another through the fixation device of FIGURES 2a to 2e.

FIGURES 4a-c are schematic illustrations of the process in which the cap is fixed to the aerosol canister.

### Detailed Description of the Exemplary Embodiment of the Invention

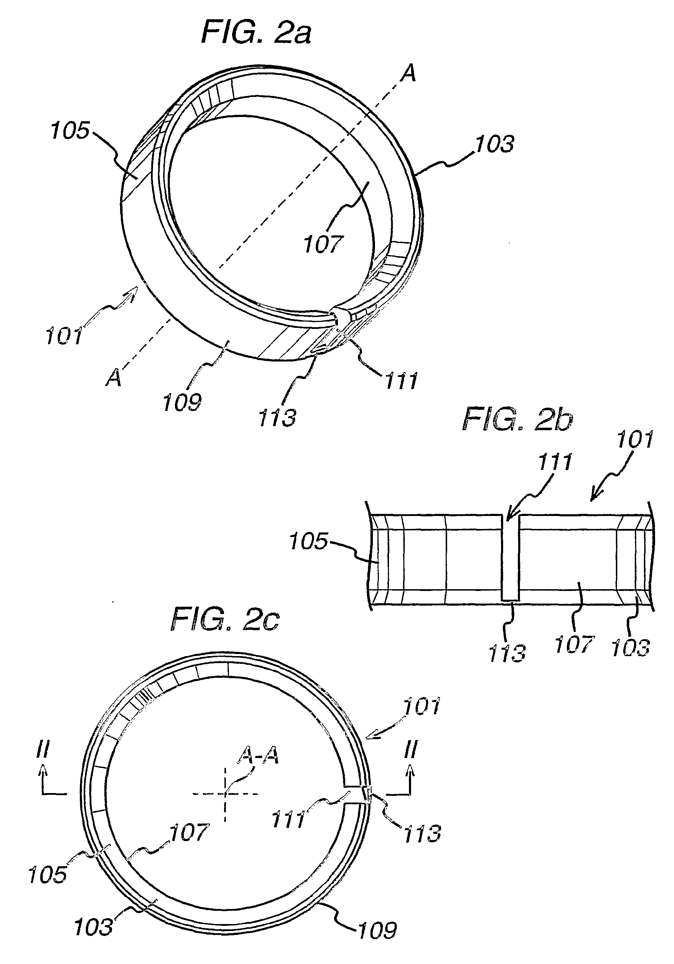

In FIGURES 2a to 2d there is shown a fixation device 101 in accordance with the present invention in its manufactured state. The fixation device 101 consists of a resilient, polypropylene ring-like body 103 defined by a circumferential wall 105 which is formed about a longitudinal axis A-A and which has an inner circumferential surface 107 and an outer circumferential surface 109 radially spaced from the inner circumferential surface 107.

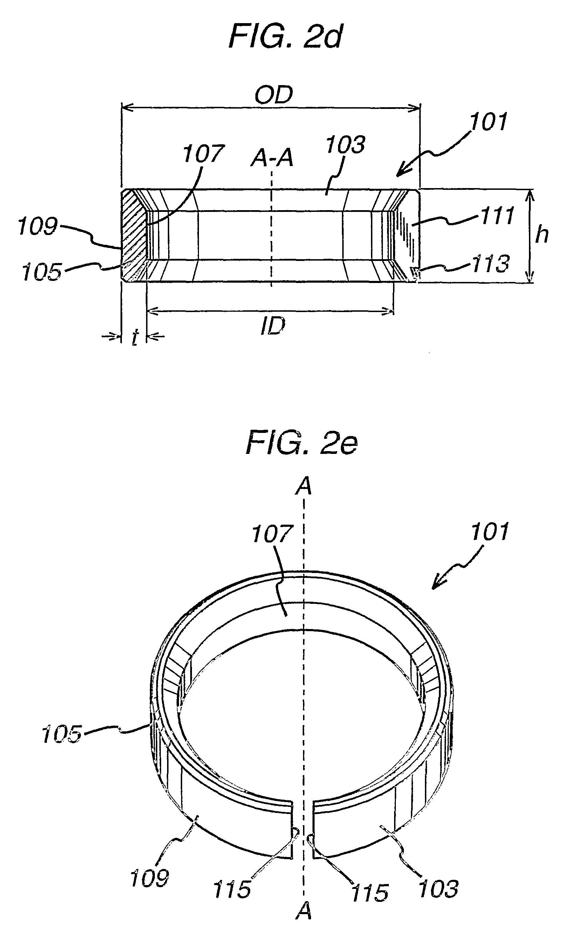

As shown in FIGURE 2d, the inner circumferential surface 107 is chamfered, whereas the outer circumferential surface 109 is planar over most of its extent. In this instance, this gives the body 103 an axial cross section of generally trapezium shape. By providing the body 103 with a cross-sectional shape which is symmetrical about an axis which is transverse to the longitudinal axis, as in this instance, allows the fixation device 101 to be used either way up, thereby aiding the assembly process, as will be more fully appreciated after reading the full description of this exemplary embodiment.

The body 103 has an outer diameter OD of about 16.5mm and an inner diameter ID of about 13.7mm. The body 103 thus has a wall thickness t of about 1.4mm. The wall 105 has a height h of about 5.2mm.

The body 103 is integrally formed with a weakened zone 111 in the form of an axial notch in the circumferential wall 105. This can be achieved where the body 103 is, for example, made by a moulding process, for instance an injection moulding process. This gives the circumferential wall 105 a circumferential zone 113 of reduced axial dimension (i.e. height).

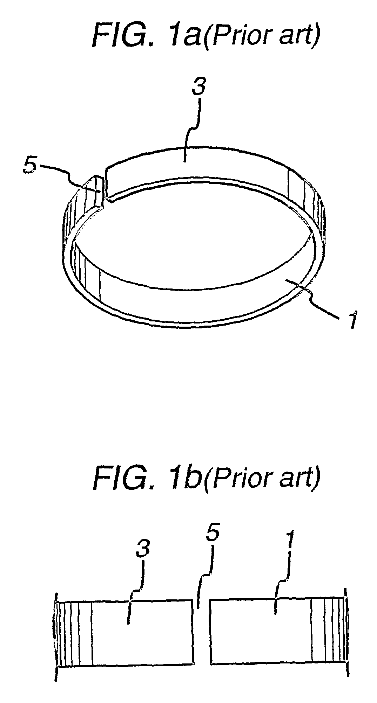

The circumferential zone 113 can be broken by applying a radial force to the circumferential wall 105. As shown in FIGURE 2e, this results in an axial split 115 being irreversibly formed in the body 103 to give the body 103 a use state having a generally C-shape, as in the prior art fixation device 1 described previously with reference to FIGURES 1 a and 1b.

However, in contrast to the prior art device 1, the device 101 of the invention does not suffer from the problem of entanglement with other devices on a production line since the body 103 has an endless angular extent on formation thereof.

Recalling that the body 103 is resilient, the body 103 in its use state is biased by a radially-inwardly directed biasing force to the radially contracted configuration shown in FIGURE 2e. However, the axial split 115 enables the body 103 to be radially expanded from the contracted radial configuration on application of a radially outward force which is greater than the biasing force. However, when the radially outward force is removed, or reduced to be less than the biasing force, the biasing force causes the body 103 to return to its contracted rest configuration. As will be appreciated, this allows the fixation device 101, in its use state, to be clamped onto an article having an outer diameter which is greater than the inner diameter ID of the body 103. In other words, a custom fit is achieved.

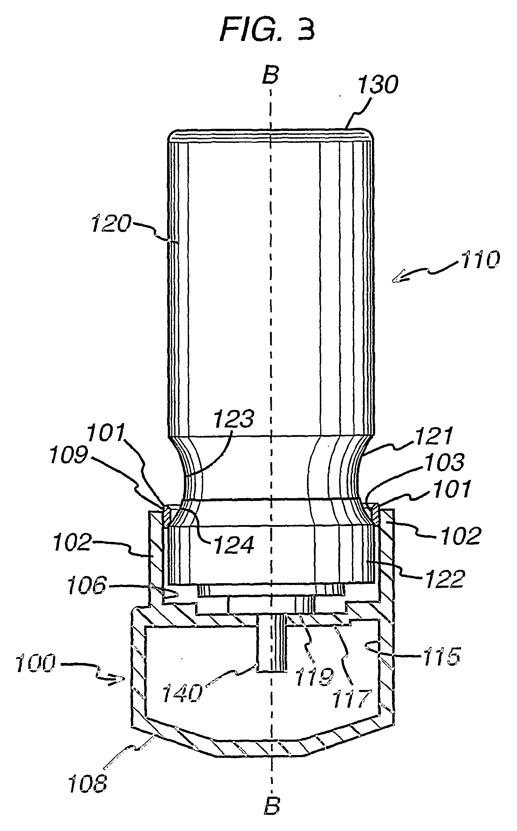

Referring now to FIGURE 3, the fixation device 101 is shown interposed between a generally cylindrical cap accessory 100 and a generally cylindrical aerosol canister 110 to fix the accessory 100 and the canister 110 together.

The aerosol canister 110 comprises as component parts thereof a container 120 containing a pressurised fluid having a closed base end 130, an open end (not shown) and a neck region 121 beneath the open end, a ferrule 122 closing the open end of the container 120, and a valve mechanism (not shown) supported in the container 120 and including an outlet valve stem 140 which projects through the ferrule 122. By displacing the valve stem 140 into the container 120 a dose of the fluid is dispensed from the aerosol canister 110 through the stem 140. The valve mechanism may be a metering valve mechanism whereby a metered dose of the fluid is dispensed by the outlet 140 on depression thereof relative to the container 120.

The ferrule 122 is secured over the open end of the container 120 by crimping the ferrule 122 into the re-entrant or flaring surface 123 presented by the neck region 121. Thus, the ferrule 122 has a crimped region 124 in the neck region 121 which is caused to adopt the profile of the neck region 121. In other words, the crimped region 124 flares radially outwardly in the direction of the business (outlet) end of the aerosol canister 110.

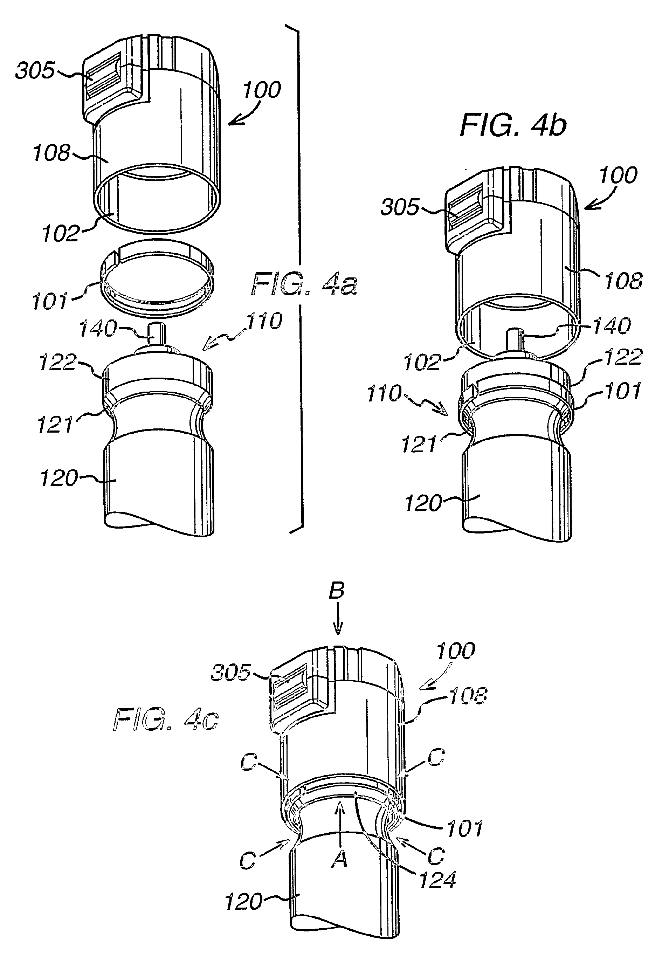

The cap accessory 100 in this non-limiting embodiment takes the form of an actuation indicator device which indicates the dispensing of the fluid from the aerosol canister 110. The actuation indicator device 100 has a housing 108, of the same material as the fixation device 101, which defines an internal compartment 115 in which is housed an indexing mechanism (not shown) which is indexed on each actuation of the aerosol canister 110 to advance a display, which has indicia to indicate the dispensing of the fluid, in a window 305 of the housing 108 (see FIGURE 4a). The display may be a numeric display which is advanced on each actuation cycle to show the number of doses of the fluid which have been dispensed or still remain. Such an actuation indicator device is disclosed in WO-A-9856444 *supra* and International patent application No. PCT/EP03/06466.

As shown in FIGURE 3, the housing 108 has a tubular sleeve or skirt 102 which is sized to slidingly receive the business end of the aerosol canister 110 along a longitudinal axis B-B. The housing 108 further has a lateral wall 117 which bounds the inner ends of the compartment 115 and the skirt 102. The lateral wall 117 delimits the sliding travel of the business end of the aerosol canister 110 into the skirt 102 by abutting with a transverse end surface 119 of the ferrule 122 at the business end. It will be seen that the length of the skirt 102 is sized so as to be co-extensive with the flaring surface 123 of the neck region 121 of the aerosol canister 110, and hence with the flared crimp region 124 of the ferrule 122, when the lateral wall 117 abuts the ferrule end surface 119.

The body 103 of the fixation device 101 is secured around the crimped region 124 of the ferrule 122, In this regard, the body 103 of the fixation device 101 has an inner diameter ID in the rest condition of its use state which is smaller than the outer diameter of the crimped region 124 of the ferrule 122. The body 103 is thus radially expanded on the crimped region 124 of the ferrule 122 so that the inner surface 107 of the body 103 is affixed to the crimped region 124. The skirt 102 of the housing 108, on the other hand, has an inner longitudinal surface 106 which engages the outer circumferential surface 109 of the body 103 of the fixation device 101. Thus the fixation device 101 is wedged between the inner longitudinal surface 106 of the skirt 102 and the flaring outer surface of the crimped region 124 of the ferrule 122.

The fixation device 101 is welded to the skirt 102 by application of ultrasound energy, e.g. by a series of spot welds. Two sonitrodes (not shown) are generally employed in the welding process, each sonitrode having three pins which are pushed into contact with the skirt 102. Energy is transferred through the sonitrode pins, causing them to vibrate and fuse the skirt 102 to the outer circumferential surface 109 of the body 103 of the fixation device 101. Alternatively, one continuous weld may be employed by moving the sonitrode head relative to the skirt 102.

It can therefore be seen that the fixation device 101 acts to fix the actuation indicator device 100 to the business end of the aerosol canister 110. Movement of the actuation indicator device 100 along the longitudinal axis B-B towards the base 130 of the aerosol container 120 is prevented by the abutment of the lateral wall 117 of the housing 108 and the ferrule end surface 119, while axial movement in the opposite direction is prevented by the fixation device 101 as the fixation device 101 is welded to the skirt 102 and wedged against the crimped region 124 of the ferrule 122 which flares radially outwardly in that direction.

FIGURES 4a-c illustrate the process whereby the fixation device 101 is fitted around the neck 121 of the aerosol container 120 and welded to tubular skirt 102 of the housing 108.

FIGURE 4a is an exploded view showing the fixation device 101 in its use state positioned between the canister 110 and the actuation indicator device 100. FIGURE 4b shows the fixation device 101 slipped around the neck 121 of the canister 110. This is done by opening the fixation device 101, sliding it over the business end of the container 120 and then allowing the return force of the body 103 to close the fixation device 101 onto the neck 121. As shown in FIGURE 4c, the fixation device 101 is slid over the canister 110 in the direction of arrow A thereby causing the body 103 to radially expand due to the interaction of the chambered inner circumferential surface 107 of the body 103 with the flaring surface of the neck region 121 and the crimped ferrule region 124. Meanwhile, the housing 108 is positioned over the business end of the canister 110 by being pressed down in the direction of arrow B. In this way, the lateral wall 117 of the housing 108 abuts the ferrule end surface 119 and the fixation device 101 is wedged between the skirt 102 and the crimped ferrule region 124.

The fixation device 101 is then joined to the skirt 102 by ultrasonic welding at the points indicated by arrows C, thereby securing the housing 108 to the canister 110.

The aerosol canister 110 may be for dispensing metered doses of a medicament in which case the container contains a pressurised medicament fluid, for instance a medicament in a liquified gas propellant, e.g. a hydrofluoroalkane (HFA) propellant such as HFA-134a or HFA-227. The aerosol canister may be for use in an inhalation device, in which case the medicament may be for the treatment of respiratory disorders such as asthma and chronic obstructive pulmonary disease. Appropriate medicaments may thus be selected from, for example, analgesics, e.g., codeine, dihydromorphine, ergotamine, fentanyl or morphine; anginal preparations, e.g., diitiazem ; antiallergics, e.g., cromoglycate (e.g. s the sodium salt), ketotifen or nedocromil (e.g. as the sodium salt) ; antiinfectives e.g., cephalosporins, penicillins, streptomycin, sulphonamides, tetracyclines and pentamidine; antihistamines, e.g., methapyrilene ; anti-inflammatories, e.g., beclomethasone (e.g. as the dipropionate ester), fluticasone (e.g. as the propionate ester), flunisolide, budesonide, rofleponide, mometasone e.g. as the furoate ester), ciclesonide, triamcinolone (e.g. as the acetonide) or 6a, 9a- difluoro-11 a-hydroxy-16a-methyl-3-oxo-17a-propionyloxy-androsta-1, 4-diene-1 7p-carbothioic acid S- (2-oxo-tetrahydro-furan-3-yl) ester; antitussives, e.g., noscapine; bronchodilators, e.g., albuterol (e.g. as free base or sulphate), salmeterol (e.g. as xinafoate), ephedrine, adrenaline, fenoterol (e.g. as hydrobromide), formoterol (e.g. as fumarate), isoprenaline, metaproterenol, phenylephrine, phenylpropanolamine, pirbuterol (e.g. as acetate), reproterol (e.g. as hydrochloride), rimiterol, terbutaline (e.g. as sulphate), isoetharine, tulobuterol or 4-hydroxy-7-[2-[[2-[[3- (2-phenylethoxy) propyl]sulfonyl]ethyl]amino]ethyl-2(3H)-benzothiazolone ; adenosine 2a agonists, e.g. 2R, 3R, 4S, 5R)-2-[6-Amino-2-(1 S-hydroxymethyl-2-phenyl-ethylamino)-purin-9-yl)-5-(2-ethyl-2H-tetrazol-5-yl)-tetrahydro-furan-3,4-diol (e.g. as maleate) ; a4 integrin inhibitors e.g. (2S)-3-[4-({[4-(aminocarbonyl)-1-piperidinyl] carbonyl}oxy)phenyl]-2-[ ((2S)-4-methyl-2-{[2-(2-methylphenoxy)acetyl]amino}pentanoyl) amino] propanoic acid (e.g. as free acid or potassium salt), diuretics, e.g., amiloride ; anticholinergics, e.g., ipratropium (e.g. as bromide), tiotropium, atropine or oxitropium; hormones, e.g., cortisone, hydrocortisone or prednisolone ; xanthines, e.g., aminophylline, choline theophyllinate, lysine theophyllinate or theophylline ; therapeutic proteins and peptides, e.g., insulin or glucagon ; vaccines, diagnostics and gene therapies. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament and/or to minimise the solubility of the medicament in the propellant. Preferred medicaments are selected from albuterol, salmeterol, fluticasone propionate and beclomethasone dipropionate and salts or solvates thereof, e.g., the sulphate of albuterol and the xinafoate of salmeterol.

Medicaments can also be delivered in combinations. Preferred formulations containing combinations of active ingredients contain salbutamol (e.g., as the free base or the sulphate salt) or salmeterol (e.g. as the xinafoate salt) or formoterol (e.g. as the fumarate salt) in combination with an anti-inflammatory steroid such as a beclomethasone ester (e.g., the dipropionate) or a fluticasone ester (e.g., the propionate) or budesonide. A particularly preferred combination is a combination of fluticasone propionate and salmeterol, or a salt thereof (particularly the xinafoate salt). A further combination of particular interest is budesonide and formoterol (e.g. as the fumarate salt).

It will be understood that the use of the terms "generally" and "about" as a prefix for certain parameters, for instance geometrical profiles and numerical values, is meant to include the exact parameter.

## Claims

1. A fixation device (101) which is for fixing together a first part (110) of a multi-part assembly to a second part (100) of the assembly, the device having a ring-like body (103), **characterised in that** the ring-like body is in a manufactured state, in which the body is manufactured with an endless angular extent and a weakened zone (113) therein, and that the weakened zone is adapted such that the ring-like body is able to be split (115) at the weakened zone to move the ring-like body from the manufactured state to a use state.

2. The device of claim 1 in which the fixation device consists of the ring-like body.

3. The device of claim 1 or 2 in which the weakened zone is a structural discontinuity in the body.

4. The device of claim 1, 2 or 3 in which the split allows the body to be radially expanded when in its use state.

5. The device of claim 4 in which the body is reversibly radially expandable when in its use state.

6. A system comprising a first part (110) of a multi-part assembly, a second part (100) of the assembly and a fixation device (101) according to any one of claims 1 to 5 for fixing the first and second parts together.

7. A system according to claim 6 in which the fixation device is in its use state interposed between the first and second parts to fix them together.

8. The system of claim 7 in which the fixation device is wedged between the first and second parts.

9. The system of claim 7 or 8 in which the fixation device is interposed between an outer surface (124) of the first part and an inner surface (106) of the second part.

10. The system of claim 9 in which one of the surfaces is a re-entrant surface.

11. The system of claim 10 in which the re-entrant surface is the outer surface of the first part.

12. The system of claim 9, 10 or 11 in which the inner surface is presented by a skirt of the second part which extends about the outer surface of the first part.

13. The system of any one of claims 7 to 12 in which the first part has a longitudinal axis and the fixation device prevents removal of the second part from the first part in a first axial direction.

14. The system of claim 13 in which the first and second parts each have an abutment surface in abutting relation to prevent the second part being removed from the first part in a second axial direction.

15. The system of any one of claims 6 to 14 in which the multi-part assembly is a product dispenser with the first part a product container.

16. The system of claim 15 in which the second part is an accessory of the dispenser.

17. The system of any one of claims 7 to 16 in which the first part has a longitudinal axis (B-B), a lateral end surface (119), and a longitudinal side surface (121,124) which extends towards the end surface in a first axial direction and which has a profile which tapers laterally outwardly in the first axial direction, and the second part has a longitudinal axis (B-B), a lateral surface (117) and a longitudinal skirt (102), wherein the first and second parts are able to be assembled with the axes aligned, the respective lateral surfaces in bearing relation and the skirt spaced laterally from the tapered profile of the longitudinal surface of the first part, and wherein the body (103) of the fixation device is adapted in use to be wedged in a radially expanded condition in the space between the skirt and the tapered profile.

18. The system of claim 17 in which the body of the fixation device is conjoined to the skirt and/or the tapered profile.

19. A method of fixing a first part (110) of a multi-part assembly to a second part (100) thereof which comprises the steps of providing a fixation device (101) according to any one of claims 1 to 5, forming an axial split in the body at the weakened zone and interposing the fixation device between the first and second parts such that the first and second parts are fixed together through the fixation device.

## Patentansprüche

1. Befestigungsvorrichtung (101) zum aneinander Befestigen eines ersten Teils (110) einer mehrteiligen Anordnung an einem zweiten Teil (100) der Anordnung, wobei die Vorrichtung einen ringartigen Körper (103) aufweist, **dadurch gekennzeichnet, dass** der ringartige Körper in einem Herstellungszustand ist, in dem der Körper mit einer Endloswinkel-Erstreckung und einem schwachen Bereich (113) in ihm hergestellt ist, und dass der schwache Bereich derart ausgebildet ist, dass der ringartige Körper an dem schwachen Bereich gespalten (115) werden kann, um den ringartigen Körper aus dem Herstellungszustand in einen Nutzungszustand zu bewegen.

2. Vorrichtung nach Anspruch 1, wobei die Befestigungsvorrichtung aus dem ringartigen Körper besteht.

3. Vorrichtung nach Anspruch 1 oder 2, bei welcher der schwache Bereich eine strukturelle Unstetigkeit in dem Körper ist.

4. Vorrichtung nach Anspruch 1, 2 oder 3, bei welcher der Spalt ermöglicht, dass sich der Körper radial aufweitet, wenn er in seinem Nutzungszustand ist.

5. Vorrichtung nach Anspruch 4, bei welcher der Körper reversibel radial aufweitbar ist, wenn er in seinem Nutzungszustand ist.

6. System mit einem ersten Teil (110) einer mehrteiligen Anordnung, einem zweiten Teil (100) der Anordnung und einer Befestigungsvorrichtung (101) nach einem der Ansprüche 1 bis 5 zum Befestigen des ersten und des zweiten Teils aneinander.

7. System nach Anspruch 6, bei dem die Befestigungsvorrichtung in ihrem Nutzungszustand zwischen dem ersten und dem zweiten Teil angeordnet ist, um sie zusammen zu befestigen.

8. System nach Anspruch 7, bei dem die Befestigungsvorrichtung zwischen dem ersten und dem zweiten Teil festgekeilt ist.

9. System nach Anspruch 7 oder 8, bei dem die Befestigungsvorrichtung zwischen einer äußeren Oberfläche (124) des ersten Teils und einer inneren Oberfläche (106) des zweiten Teils angeordnet ist.

10. System nach Anspruch 9, bei dem eine der Oberflächen eine in sich zurückkehrende Oberfläche ist.

11. System nach Anspruch 10, bei dem die in sich zurückkehrende Oberfläche die äußere Oberfläche des ersten Teils ist.

12. System nach Anspruch 9, 10 oder 11, bei dem die innere Oberfläche durch einen Kragen des zweiten Teils dargestellt ist, der sich um die äußere Oberfläche des ersten Teils erstreckt.

13. System nach einem der Ansprüche 7 bis 12, bei dem das erste Teil eine Längsachse aufweist, und die Befestigungsvorrichtung ein Entfernen des zweiten Teils von dem ersten Teil in einer ersten axialen Richtung verhindert.

14. System nach Anspruch 13, bei dem das erste und das zweite Teil jeweils eine Anstoßoberfläche in einer anstoßenden Beziehung aufweisen, um zu verhindern, dass das zweite Teil von dem ersten Teil in einer zweiten axialen Richtung entfernt wird.

15. System nach einem der Ansprüche 6 bis 14, bei dem die mehrteilige Anordnung ein Produktspender ist, wobei das erste Teil ein Produktbehälter ist.

16. System nach Anspruch 15, bei dem das zweite Teil ein Zubehör des Spenders ist.

17. System nach einem der Ansprüche 7 bis 16, bei dem das erste Teil eine Längsachse (B-B) aufweist, eine seitliche Endoberfläche (119) und eine Längsseitenoberfläche (121, 124), die sich zu der Endoberfläche in eine erste axiale Richtung hin erstreckt, und die ein Profil aufweist, das sich seitlich nach außen in der ersten axialen Richtung verjüngt, und wobei das zweite Teil eine Längsachse (B-B) aufweist, eine seitliche Oberfläche (117) und einen Längskragen (102), wobei das erste und das zweite Teil mit den Achsen ausgerichtet zusammengebaut werden können, wobei die jeweiligen Seitenoberflächen in einer tragenden Beziehung sind und der Kragen seitlich von dem sich verjüngenden Profil der Längsoberfläche des ersten Teils beabstandet ist, und wobei der Körper (103) der Befestigungsvorrichtung geeignet ist, bei der Nutzung in einem radial aufgeweiteten Zustand in dem Raum zwischen dem Kragen und dem sich verjüngenden Profil festgekeilt zu werden.

18. System nach Anspruch 17, bei dem der Körper der Befestigungsvorrichtung mit dem Kragen und/oder dem sich verjüngenden Profil verbunden ist.

19. Verfahren zur Befestigung eines ersten Teils (110) einer mehrteiligen Anordnung an einem zweiten Teil (110) von ihr, das die Schritte des Vorsehens einer Befestigungsvorrichtung (101) nach einem der Ansprüche 1 bis 5, Ausbilden eines axialen Spalts in dem Körper an dem schwachen Bereich, und Anordnen der Befestigungsvorrichtung zwischen dem ersten und dem zweiten Teil umfasst, derart, dass das erste und das zweite Teil durch die Befestigungsvorrichtung aneinander befestigt werden.

## Revendications

1. Dispositif de fixation (101) prévu pour fixer mutuellement un premier élément (110) d'un ensemble multi-éléments à un second élément (100) dudit ensemble, le dispositif comprenant un corps de forme annulaire (103), **caractérisé en ce que** le corps de forme annulaire est fourni dans une configuration de sortie d'usine, dans laquelle le corps est produit de sorte à présenter une extension angulaire sans fin et une zone de faible résistance (113) ménagée dans cette dernière, et **en ce que** la zone de faible résistance est adaptée de telle manière que le corps de forme annulaire puisse être fendu (115) à l'endroit de la zone de faible résistance afin de faire passer le corps de forme annulaire de la configuration de sortie d'usine à une configuration d'utilisation.

2. Dispositif selon la revendication 1, dans lequel le dispositif de fixation consiste en un corps de forme annulaire.

3. Dispositif selon la revendication 1 ou 2, dans lequel la zone de faible résistance correspond à une discontinuité structurelle dans le corps.

4. Dispositif selon la revendication 1, 2 ou 3, dans lequel la fente permet au corps d'être dilaté radialement lorsqu'il est dans sa configuration d'utilisation.

5. Dispositif selon la revendication 4, dans lequel le corps peut être dilaté radialement de façon réversible lorsqu'il est dans sa configuration d'utilisation.

6. Système comprenant un premier élément (110) d'un ensemble multi-éléments, un second élément (100) dudit ensemble et un dispositif de fixation (101) conforme à l'une quelconque des revendications 1 à 5 prévu pour fixer les premier et second éléments mutuellement.

7. Système selon la revendication 6, dans lequel le dispositif de fixation est, lorsqu'il est dans sa configuration d'utilisation, interposé entre les premier et second éléments de telle manière à les fixer mutuellement.

8. Système selon la revendication 7, dans lequel le dispositif de fixation est coincé entre les premier et second éléments.

9. Système selon la revendication 7 ou 8, dans lequel le dispositif de fixation est interposé entre une surface extérieure (124) du premier élément et une surface intérieure (106) du second élément.

10. Système selon la revendication 9, dans lequel une des surfaces est une surface rentrante.

11. Système selon la revendication 10, dans lequel la surface rentrante est la surface extérieure du premier élément.

12. Système selon la revendication 9, 10 ou 11, dans lequel la surface intérieure est constituée par une jupe du second élément qui s'étend autour de la surface extérieure du premier élément.

13. Système selon l'une quelconque des revendications 7 à 12, dans lequel le premier élément présente un axe longitudinal et le dispositif de fixation empêche le retrait du second élément depuis le premier élément dans une première direction axiale.

14. Système selon la revendication 13, dans lequel les premier et second éléments présentent tous deux une surface de butée en relation de butée l'une sur l'autre de manière à empêcher le retrait du second élément depuis le premier élément dans une seconde direction axiale.

15. Système selon l'une quelconque des revendications 6 à 14, dans lequel l'ensemble multi-éléments est un distributeur de produit, le premier élément étant un récipient à produit.

16. Système selon la revendication 15, dans lequel le second élément est un accessoire du distributeur.

17. Système selon l'une quelconque des revendications 7 à 16, dans lequel le premier élément présente un axe longitudinal (B-B), une surface d'extrémité latérale (119), et une surface latérale s'étendant sur la longueur (121, 124) qui s'étend en direction de la surface d'extrémité dans une première direction axiale et qui présente un profil qui converge latéralement vers l'extérieur dans la première direction axiale, et le second élément présente un axe longitudinal (B-B), une surface latérale (117) et une jupe longitudinale (102), dans lequel les premier et second éléments peuvent être assemblés avec alignement des axes, mise en butée mutuelle des surfaces latérales respectives, et espacement latéral de la jupe par rapport au profil convergent de la surface longitudinale du premier élément, et dans lequel le corps (103) du dispositif de fixation est adapté en utilisation pour être coincé dans un état d'expansion radiale dans l'espace défini entre la jupe et le profil convergent.

18. Système selon la revendication 17, dans lequel le corps du dispositif de fixation est solidaire de la jupe et/ou du profil convergent.

19. Procédé de fixation d'un premier élément (110) d'un ensemble multi-éléments (100) à un second élément de ce dernier, comprenant les étapes suivantes : procurer un dispositif de fixation (101) conforme à l'une quelconque des revendications 1 à 5, ménager une fente axiale dans le corps à l'endroit de la zone de faible résistance, et interposer le dispositif de fixation entre les premier et second éléments de telle manière que les premier et second éléments soient fixés mutuellement au moyen du dispositif de fixation.
